# EUROPÄISCHE PATENTSCHRIFT

(11) **EP 0 509 322 B1**
(45) Veröffentlichungstag und Bekanntmachung des Hinweises auf die Patenterteilung: **27.07.1994**
(21) Anmeldenummer: 92105640.4
(22) Anmeldetag: 02.04.1992
(51) Int. Cl.: F16B 7/04, F16B 2/04, A61B 17/60

(54) **Verbindungseinrichtung zum verstellbaren Verbinden eines ersten mit einem zweiten Konstruktionselement, insbesondere von Rohren oder Stäben für eine Fixationsvorrichtung**
Connecting device for the adjustable joint of a first construction element with a second, in particular for tubes or bars for a fixing device
Dispositif de connexion pour la connexion réglable d'un premier élément de construction avec un deuxième, en particulier de tubes ou barres pour un dispositif de fixation

(30) Priorität: 16.04.1991 CH 1124/91
(43) Veröffentlichungstag der Anmeldung: 21.10.1992
(73) Patentinhaber: Synthes AG, Chur, CH-7002 Chur (CH)
(72) Erfinder: Schläpfer, Johannes Fridolin, Dr., CH-8750 Glarus (CH); Hess, Martin, CH-4434 Hölstein (CH)
(74) Vertreter: Lusuardi, Werther Giovanni, Dr.

(56) Entgegenhaltungen:
- US-A- 1 997 466
- US-A- 4 244 360
- US-A- 4 865 484

## Beschreibung

Die Erfindung betrifft eine Verbindungseinrichtung zum verstellbaren Verbinden eines ersten mit einem zweiten Konstruktionselement, insbesondere von Rohren oder Stäben für eine Fixationsvorrichtung, mit einem eine zylindrische Bohrung aufweisenden äusseren Element und mindestens einem in der Bohrung wenigstens axial verschiebbaren inneren zylindrischen Element, welche eine, vorzugsweise zwei gemeinsame quer zur Achsrichtung gebildete Durchgangsöffnungen zur Aufnahme eines Konstruktionselementes aufweisen, die darin eingeführt mittels den durch mindestens ein Verspannungsmittel axial gegeneinander verschiebbaren Elementen festklemmbar sind (siehe zum Beispiel die US-A-1997466).

Eine bekannte Verbindungseinrichtung für eine osteosynthetische externe Fixationsvorrichtung besteht einerseits aus zwei Spannbacken und einer diese durchdringenden Schraube sowie andererseits aus einer mit der genannten Schraube verbundener Oese, aus einem letztere umfassenden U-förmigen Gegenstück und aus einer dieses Gegenstück durchdringenden und an der Oese befestigten Schraube. Vorteilhaft wird mit den Spannbacken dieser Verbindungseinrichtung bspw. eine sogenannte Schanzsche Schraube befestigt, während mit dem gegenüberliegenden Teil der Verbindungseinrichtung ein Stab, bspw. ein Kohlefaserstab festspannbar ist, welcher bspw. bei einer Knochenfraktur annähernd parallel zu einem gebrochenen Knochen angeordnet ist und über die genannten Verbindungseinrichtungen mit mehreren, beidseitig zur Bruchstelle in den Knochen eingeschraubten Schanzschen Schrauben verbunden ist; damit ist die angestrebte externe Fixation des Knochens erreicht. Hierbei ist sehr wichtig, dass diese Verbindungseinrichtung sehr einfach zu handhaben ist und ohne übermässsigen Kraftaufwand trotzdem eine sehr starre Fixation bewirkt wird, so dass bei Erschütterungen oder anderen Fremdeinwirkungen die Knochenteile sich gegenseitig nicht bewegen und die Fixation auch nach einiger Zeit ihr Starrheit beibehält. Bei der beschriebenen bekannten Verbindungseinrichtung hat sich in der Praxis gezeigt, dass gerade im Hinblick auf die Beibehaltung seiner Spannkräfte über eine längere Zeitdauer diese etwas nachlassen können und damit nicht immer optimale Bedingungen vorliegen.

Die Aufgabe der vorliegenden Erfindung besteht darin, eine gattungsgemässe Verbindungseinrichtung derart weiterzubilden, dass mit ihr bei vorausgesetzter einfacher Ausführung und flexibler Handhabung eine sichere und über längere Zeitdauer hinhaltende starre Verbindung erzielt wird.

Erfindungsgemäss ist die Aufgabe dadurch gelöst, dass das mittels dem Verspannungsmittel axial zum äusseren Element verschiebbare innere Element mit wenigstens einer Ausnehmung versehen ist, vermittels derer es beim Verschieben durch das eingeführte Konstruktionselement elastisch deformierbar und damit - nebst der unmittelbaren Verklemmung der Stäbe - gegen das äussere Element zusätzlich verspannbar ist. Damit sind die Bedingungen gemäss der obgenannten Aufgabe in idealer Weise erfüllt, denn es ergibt sich mit einer solchen Anordnung im inneren Element ein zusätzliches Blockieren zwischen diesem und dem äusseren Element und daher eine trotz vereinfachter Ausführung und leichter Bedienbarkeit sehr wirksame und zuverlässige stabile Fixation.

Vorteilhaft sind die miteinander zur Anlage kommenden Flächen des inneren und äusseren Elementes mit strukturierten oder verzahnten Oberflächen versehen, damit die Verspannung dieser Elemente kraft- resp. formschlüssig ist und dadurch eine noch grössere Sicherheit gegen Verdrehung dieser beiden Elemente erzielt wird. Das innere Element hat dabei zum äusseren einen solchen Aussendurchmesser, dass es entweder in unverspanntem Zustand drehbar oder aber bereits annähernd formschlüssig im äusseren Element gehalten ist.

Das Verspannungsmittel kann eine Mutter umfassen, die auf ein am inneren Element korrespondierenden Gewindeteil aufschraubbar ist und dabei mit dem äusseren Element zur Anlage kommt. Es kann aber auch eine Stellschraube umfassen, welche in eine Gewindebohrung eines inneren Elementes einschraubbar ist und mit einem darin eingeführten Konstruktionselement zur Anlage kommt.

Die für die Konstruktionselemente vorgesehenen Durchgangsöffnungen sind als Bohrungen und/oder als Schlitze in den Elementen ausgebildet. Dabei sollen diese Durchgangsöffnungen eine sektorielle Beweglichkeit des darin eingeführten Konstruktionselementes innerhalb eines Winkelbereiches gestatten.

Weitere vorteilhafte Verbindungseinrichtungen sind in den nachfolgenden Ausführungsbeispielen anhand der Zeichnung näher erläutert. Es zeigt:
- Fig.1: eine perspektivische Ansicht einer externen Fixationsvorrichtung mit erfindungsgemässen Verbindungseinrichtungen,
- Fig.2: eine Verbindungseinrichtung nach Fig.1 perspektivisch dargestellt,
- Fig.3 und Fig.4: je eine Ausführungsvariante einer Verbindungseinrichtung im Längsschnitt,
- Fig.5: eine weitere Variante in Ansicht,
- Fig.6 und Fig.7: je eine perspektivische Ansicht zweier weiterer ähnlicher Verbindungseinrichtungen,
- Fig.8 und Fig.9: je eine perspektivische Ansicht zweier Verbindungseinrichtungen mit einem Wirbelhaken resp. einer Pedikelschraube,
- Fig.10: eine Teilansicht eines inneren Elementes einer Verbindungseinrichtung und
- Fig.11: eine weitere Variante in teilweiser Ansicht.

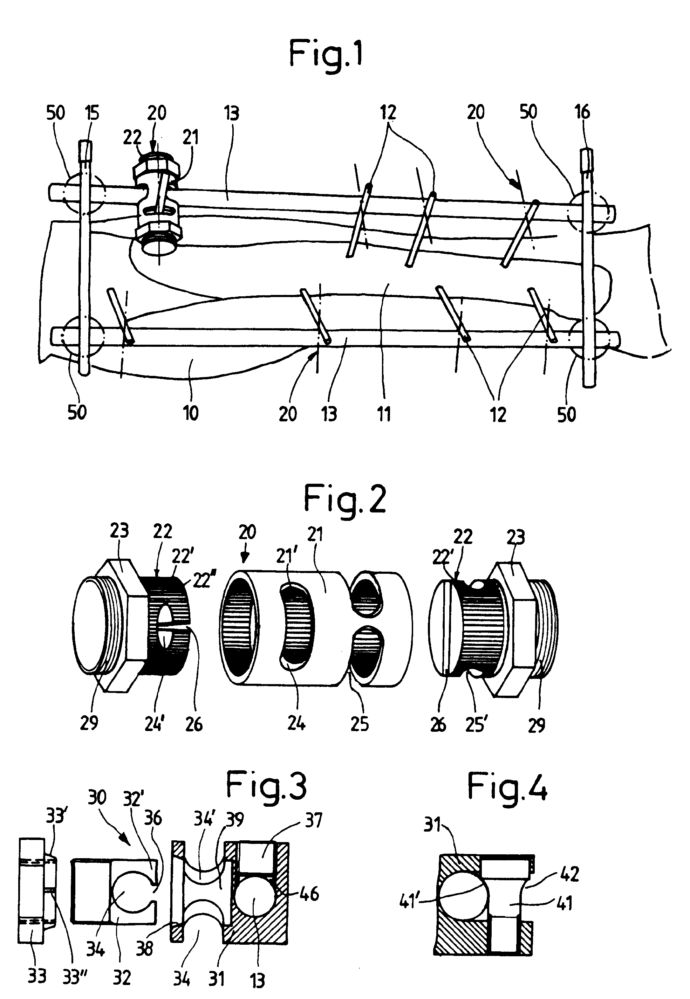

Fig.1 zeigt eine osteosynthetische externe Fixationsvorrichtung am Beispiel einer Unterschenkelfraktur an einem teilweise dargestellten Bein 10. Der Knochen (Schienbein) 11 hat entlang seiner Längsausdehnung sogenannte Schanzsche Schrauben 12 als Konstruktionselemente eingeschraubt, die in zwei Reihen à je vier Schrauben angeordnet sind und jeder ein annähernd parallel zum Knochen 11 positionierter Kohlefaserstab 13 zugeordnet ist. Mittels erfindungsgemässen Verbindungseinrichtungen 20 sind die Schrauben jeweils starr mit dem entsprechenden Kohlefaserstab 13 verbunden. Zusätzliche Verbindungsstäbe 15 und 16, die an beiden Enden der Stäbe 13 in Querrichtung angeordnet und wiederum durch erfindungsgemässe Verbindungseinrichtungen 50 mit diesen fest fixiert sind, vermitteln dem verletzten Knochen eine zusätzliche Rotationsstabilität. Anstelle der Verbindungseinrichtung 50 könnten im Prinzip auch die Einrichtungen 20 verwendet werden. Die Stäbe haben jeweils einen runden Querschnitt, sie könnten aber auch rechteckig oder andersförmig sein. Die als Kohlefaserstäbe ausgebildeten Fixationsstäbe sind zudem röntgenstrahlendurchlässig. In Fig.2 ist eine Verbindungseinrichtung 20 gezeigt, die aus einem eine zylindrische Bohrung aufweisenden äusseren Element 21, aus zwei in der Bohrung in unverspanntem Zustand längs- und drehverschiebbaren zylindrischen inneren Elementen 22 sowie aus auf diesen je einer aufgeschraubten Mutter 23 besteht. Die genannte Mutter 23 und der Gewindeteil 29 am jeweiligen inneren Element 22 bilden das Verspannungsmittel. Das äussere Element 21 hat zwei als Schlitze ausgebildete Durchgangsöffnungen 24 und 25, die quer zu seiner Längsachse und annähernd rechtwinklig zueinander angeordnet sind. Die Schlitze ermöglichen eine Einstellung der Stäbe zueinander bis zu einer Winkeldifferenz von beispielsweise 80°. Die inneren Elemente haben ihrerseits je eine zylindrische Durchgangsöffnung 24′ und 25′, die zusammen mit den Schlitzen die Durchgangsöffnungen 24 und 25 bilden, in welche jeweils ein Kohlefaserstab 13 einerseits und eine Schanzsche Schraube 12 andererseits eingeführt werden kann.

An dem mit der Durchgangsöffnung 24′, 25′ versehenen Ende 22˝ haben die inneren Elemente 22 erfindungsgemäss je eine das Ende 22˝ sowie die Oeffnung 24′, 25′ trennende Ausnehmung 26, die beim Festziehen einer Mutter 23 durch den eingeschobenen Stab erzeugten Axialdruck eine elastische radiale Aufspreizung und damit ein zusätzliches Verspannen dieses Endes 22˝ des inneren gegen das äussere Element 22, 21 bewirkt. Eine am Umfang des inneren Elementes 22 versehene Aussenverzahnung 22′ greift dabei formschlüssig in eine Innenverzahnung 21′ des äusseren Elementes 21 ein und verstärkt durch den bewirkten Formschluss wesentlich die Rotationsstabilität eines eingespannten Stabes zu einer Schraube 12. Die Achse des inneren Elementes 22, die quere Durchgangsöffnung 24′, 25′ sowie dessen Ausnehmung 26 liegen in einer Ebene. Im Prinzip könnte die von der Oeffnung 24′, 25′ ausgehende schlitzförmige Ausnehmung 26 in einem bestimmten Winkel, bspw. 30°, zur genannten Ebene angeordnet sein. Seine Breite muss jedoch kleiner als der Durchmesser der Oeffnung resp. des eingeführten Stabes sein.

Beim Verspannen zweier Stäbe 12 und 13 hat diese Verbindungseinrichtung 20 überdies den Vorteil, als die entstehenden Drehmomente beim gleichzeitigen Festziehen der Muttern 23 sich gegenseitig aufheben und dadurch beim Spannen oder Lösen nicht unerwünschte Kräfte auf die Schraube und damit auf den Knochen wirken.

Bei der Verbindungseinrichtung 30 nach der Fig.3 ist auf der einen Seite des äusseren Elementes 31 ähnlich der oben näher beschriebenen Verbindungseinrichtung 20 ein inneres zylindrisches Element 32 und eine darauf aufschraubbare Mutter 33 vorgesehen. Das innere Element 32 ist dabei in die Bohrung 39 des äusseren Elementes 31 einsteckbar und diese bilden eine quere Durchgangsöffnung 34, in die ein Stab eingeführt werden kann. Durch das axiale Verschieben des inneren Elementes 32 wird es durch eine an dessen Ende 32′ vorgesehener Ausnehmung 36 wiederum radial deformiert und verspannt sich dadurch zusätzlich im äusseren Element 31. Das äussere Element 31 weist auch ein Schlitz als Durchgangsöffnung 34′ auf, vermittels dem die Stäbe zueinander bspw. bis zu einer Winkeldifferenz von 40° einstellbar sind. Der andere Stab 13 ist in eine im äusseren Element 31 enthaltener Oeffnung 46, die zum Schlitz 34 annähernd in einem Winkel von 90° angeordnet ist, eingeschoben und wird von einer Stellschraube 37 im Element blockiert. Die Mutter 33 hat fernerhin einen mit einem Innenkonus 38 des äusseren Elementes 31 korrespondierenden konischen Ansatz 33′, durch welchen eine zusätzliche Klemmwirkung entsteht, dabei auch die Gefahr eines selbsttätigen Lösens der Mutter 33 praktisch ausgeschossen wird. Die Selbsthemmung zwischen dem inneren und dem äusseren Element 32, 31 ist durch die im konischen Ansatz 33′ vorgesehenen Längsschlitz 33˝ erhöht, denn beim Verspannen wird der konische Ansatz 33′ auf das Gewinde des inneren Elementes 32 gedrückt.

Bei der Verbindungseinrichtung nach Fig.4 ist nur gerade der eine Teil des ansich dem nach Fig.3 entsprechenden äusseren Elementes 31 gezeigt. Dieser hat anstelle der Stellschraube 37 eine neben dem Stab 13 angeordnete Schraube 41, die eine dem Stab 13 entsprechende abgerundete Klemmfläche 41′ hat, durch die der Stab 13 beim Einschrauben verklemmt wird. Eine stirnförmige Ausnehmung 42 erhöht die Klemmwirkung.

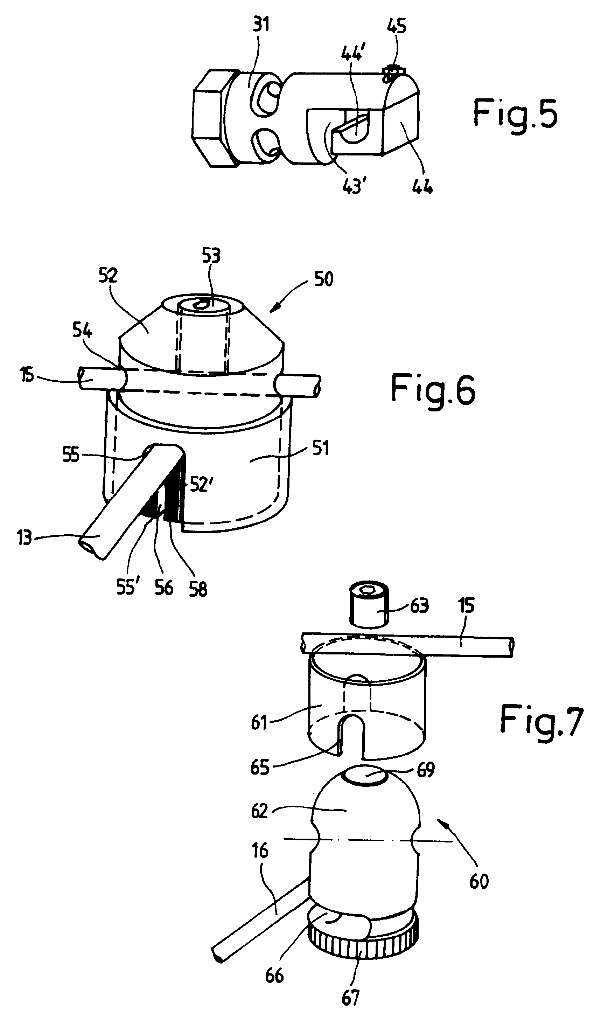

Auch die Verbindungseinrichtung nach Fig.5 unterscheidet sich von der nach Fig.3 nur nach der Art der Halterung und Verspannung des einen Stabes. Das äussere Element 31 hat an dem einen Ende eine segmentförmige Ausnehmung 43′, in der ein Klemmwinkel 44 mit einer halbrunden Aussparung 44′ vorgesehen ist, der mittels einer im Element 31 vorgesehener Schraube 45 verstellbar angeordnet ist. Ein Stab ist in die Aussparung 44′ des Klemmwinkels 44 einlegbar und wird durch Verdrehen der Schraube 45 zwischen diesen und das äussere Element 31 geklemmt.

Die Verbindungseinrichtung 50 gemäss Fig.6 setzt sich aus einem inneren, im wesentlichen zylindrischen Element 52, einer in diesem koaxial angeordneter Stellschraube 53 als Verspannmittel sowie aus einem dieses innere Element 52 umschliessenden als Ring ausgebildeten äusseren Element 51 zusammen. Das innere Element 52 hat eine quere Durchgangsöffnung 54 für einen Verbindungsstab 15 sowie eine unterhalb und rechtwinklig zu dieser vorgesehenen Durchgangsöffnung 55, in die ein Stab 13 einschiebbar ist. Das äussere Element 51 kommt beidseitig mit je einem Stab 13 bzw. 15 zur Anlage. Die Schraube 53 ist ihrerseits derart im inneren Element 52 angeordnet, dass sie beim Einschrauben mit dem Stab 15 in Anlage kommt, der seinerseits auf das anliegende äussere Element 51 und folglich auf den unteren Stab 13, der im inneren Element 52 aufliegt, wirkt. Damit lassen sich mit derselben Schraube 53 gleichzeitig beide Stäbe 13 und 15 verklemmen. Der Stab 15 liegt auf der oberen Stirnfläche, während der untere Stab 13 sich in einer nach unten offenen Aussparung des äusseren Elementes 51 befindet.

Erfindungsgemäss hat das innere Element 52 wiederum eine als Längsschlitz ausgebildete Ausnehmung 56, die von der Durchgangsöffnung 55 ausgeht und das Ende 52′ des inneren Elementes 52 trennt. Wenn beim Festspannen der beiden Stäbe 13 und 15 das Element 51 auf den unteren Stab 13 und folglich auf das innere Element 52 drückt, entsteht sinngemäss die zusätzliche Verspannkraft durch die Aufspreizung der Elemente 52. Entsprechende Verzahnungen 58 an den Elementen 52 und 51 bewirken die Erhöhung der genannten Rotationssteifigkeit.

Diese Verbindungseinrichtung 50 eignet sich gemäss Fig.1 besonders zum Verspannen der Verbindungsstäbe 15, 16 mit den Stäben 13, 14, da im Normalfall die letzteren zuerst mit den Schanzschen Schrauben 12 verbunden und anschliessend erst die Verbindungsstäbe 15, 16 fixiert werden.

Fig.7 zeigt eine Verbindungseinrichtung 60, die sich von der nach Fig.6 nur insofern unterscheidet, als ein inneres Element 62 anstelle des Längsschlitzes 56 mit einer quer zu seiner Achse angeordneten Ausnehmung 66 versehen ist. Wenn nun beim Verspannen dieser Einrichtung 60 in entsprechender Weise ausgehend von der Schraube 63 in der Gewindebohrung 69 des inneren Elementes 62 über den quer eingeführten Stab 15 und das äussere Element 61 der nachfolgende Stab 16 wiederum mit dem inneren Element 62 zur Anlage kommt, so wird auf dieses Element 62 durch dessen quere Ausnehmung 66 eine Biegekraft und damit eine elastisch wirkende Aufbiegung desselben verursacht, die das zusätzliche gegenseitige Verklemmen dieser beiden Elemente 62 und 61 bewirken. Die Ausnehmung 66, die zudem als Durchgangsöffnung dient, ist dabei so ausgebildet, dass der darin einlegbare Stab 16 zum oberen Stab 15 um einen bestimmten Winkel vestellbar ist und so eine sektorielle Bewegung gestattet. Anstelle der Stellschraube 63 könnte eine Mutter vorgesehen sein, die auf ein Aussengewinde des inneren Elementes aufschraubbar wäre.

Im übrigen kann dieser Stab 16 zuerst in diese Ausnehmung 66 eingeführt werden und dann das äussere aufs innere Element gestülpt werden, oder aber es kann in die durch die beiden Elemente 62 und 61 gebildete Durchgangsöffnung 65, 66 eingeschoben werden. Strukturierte Oberflächen 67 zumindest der miteinander zur Anlage kommenden Flächen der Elemente 61 und 62 ermöglichen eine kraftschlüssige Verspannung derselben. Bei den beschriebenen strukturierten Oberflächen könnte es sich um Einkerbungen, Aufrauhungen oder dergleichen handeln.

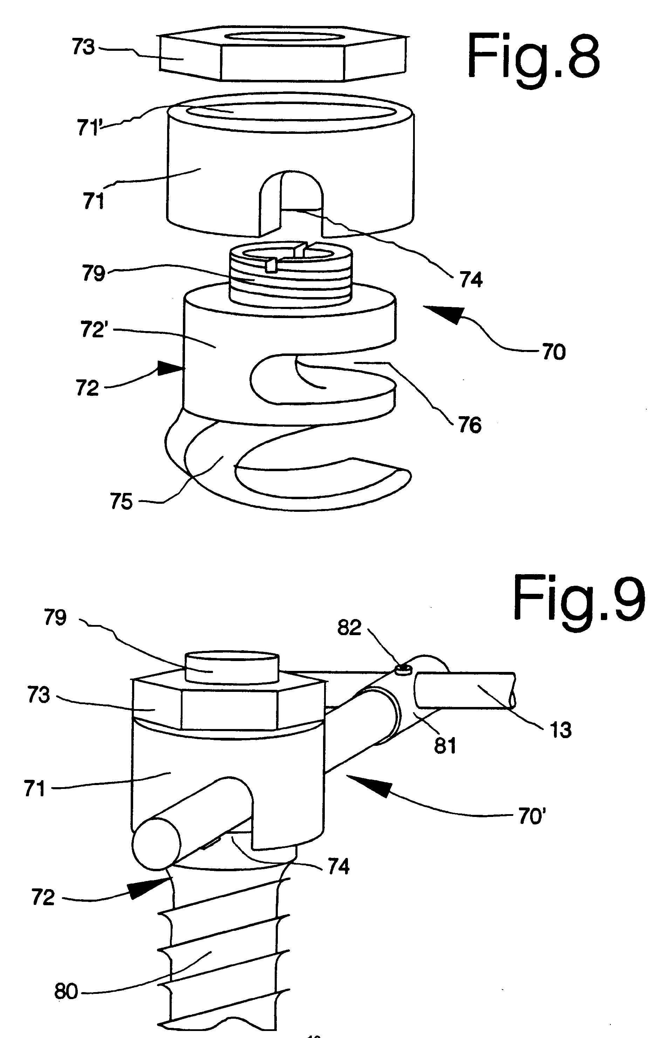

Die Verbindungseinrichtung 70 entsprechend der Fig.8 hat ein inneres Element 72, welches sich aus einem Gewindeteil 79, einem zylindrischen Teil 72′ mit einer queren Ausnehmung 76 und einem das eine Konstruktionselement bildenden Wirbelhaken 75 zusammensetzt. Wiederum wird ein eine zylindrische Bohrung 71′ enthaltendes äusseres Element 71 aufs innere Element 72 aufgesteckt und von einer auf dem Gewindeteil 79 des inneren Elementes 72 aufschraubbarer Mutter 73 gegen ein Konstruktionselement 13 gedrückt, welches in der Ausnehmung 76 des inneren Elementes 72 zur Anlage kommt und gleichsam eine Biegekraft und damit eine radiale im elastischen Bereich erfolgende Deformation des inneren Elementes 72 erzeugt. Nebst der durch Haftreibung entstehenden Verklemmung des Konstruktionselementes 13 im inneren Element 72 verspannen sich die beiden Elemente vermittels der genannten Deformation zusätzlich gegeneinander und erhöhen wie bereits hinlänglich beschrieben, die Verspannkraft der Konstruktionselemente zueinander. Beim äusseren Element 71 sind für die Durchgangsöffnung 74 zwei stirnseitige Einschlitzungen vorgesehen. Die quere Ausnehmung 76 des inneren Elementes 72, die gleichzeitig als Durchgangsöffnung für ein Konstruktionselement dient, kann auch wieder derart ausgebildet werden, dass der darin eingeführte Stab sich sektoriell bewegen kann.

Fig.9 zeigt eine Verbindungseinrichtung 70′, die sich von der nach Fig.8 nur gerade durch ein am inneren zylindrischen Element 82 angebrachter Pedikelschraube 80 als Konstruktionselement unterscheidet. Ansonsten entsprechen deren Bestandteile denjenigen von Fig.8 und sie sind daher nicht mehr im Detail beschrieben. Fernerhin ist das gezeigte Konstruktionselement als Querstab 81 ausgebildet, der eine Querbohrung aufweist, in welcher der eigentliche Verbindungsstab 13 getragen ist. Eine Stellschraube 82 im Querstab 81 dient als Verspannmittel der beiden Stäbe.

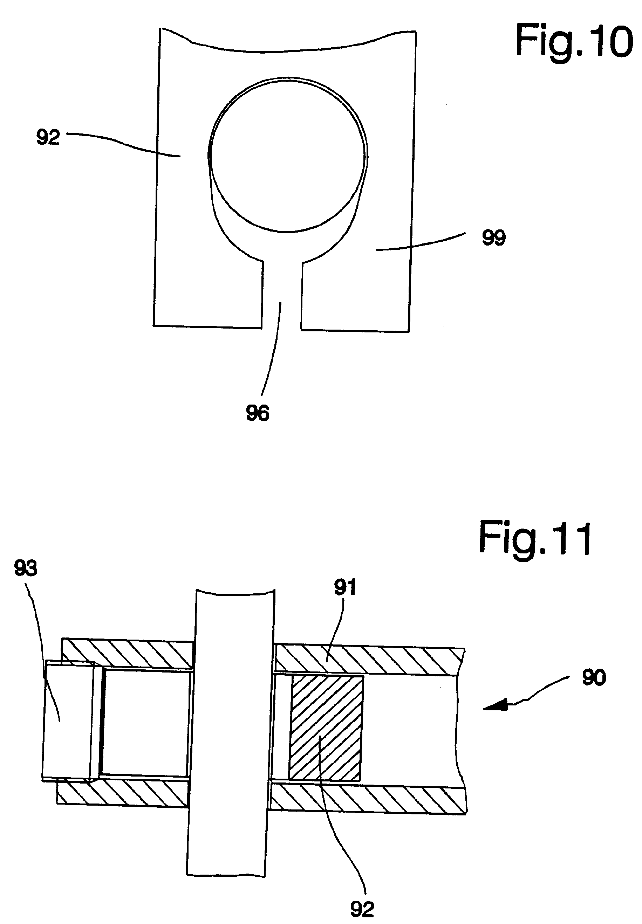

In Fig.10 ist ein inneres Element 92 mit einer Ausnehmung 96 gezeigt, das eine Doppelbohrung 99 als Durchgangsöffnung für ein Konstruktionselement aufweist. Diese Doppelbohrung 99 ist aus zwei versetzten Bohrungen gebildet, von denen diejenige gegen das Ende des inneren Elementes 92 angebrachte mit einem kleineren Durchmesser als die andere resp. der des Konstruktionselementes ausgebildet ist, dadurch sich die Aufspreizung erhöhen lässt.

Die Verbindungseinrichtung 90 nach der Fig.11 unterscheidet sich von der nach Fig.2 nur insofern, als das Verspannungsmittel anstelle der Mutter eine Stellschraube 93 umfasst, die ins äussere Element 91 einschraubbar ist und gegen das innere Element 92 drückt.

## Patentansprüche

1. Verbindungseinrichtung zum verstellbaren Verbinden eines ersten mit einem zweiten Konstruktionselement, insbesondere von Rohren oder Stäben für eine Fixationsvorrichtung, mit einem eine zylindrische Bohrung aufweisenden äusseren Element und mindestens einem in der Bohrung wenigstens axial verschiebbaren inneren zylindrischen Element, welche eine, vorzugsweise zwei gemeinsame quer zur Achsrichtung gebildete Durchgangsöffnungen zur Aufnahme eines Konstruktionselementes aufweisen, die darin eingeführt mittels den durch mindestens ein Verspannungsmittel axial gegeneinander verschiebbaren Elementen festklemmbar sind, dadurch gekennzeichnet, dass das mittels dem Verspannungsmittel (23,29,33,53,63,73) axial zum äusseren Element (21,31,51,61,71) verschiebbare innere Element (22,32,52,62,72) mit wenigstens einer Ausnehmung (26,36,56,66,76) versehen ist, vermittels derer es beim Verschieben durch das eingeführte Konstruktionselement (12,13,15,16) elastisch deformierbar und damit gegen das äussere Element (21,31,51,61,71) zusätzlich verspannbar ist.

2. Verbindungseinrichtung nach Anspruch 1, dadurch gekennzeichnet, dass die Verspannung des inneren im äusseren Element (62,61) kraftschlüssig ist, insbesondere mittels strukturierter Oberflächen (67) der miteinander zur Anlage kommenden Flächen des inneren und äusseren Elementes (62,61).

3. Verbindungseinrichtung nach Anspruch 1, dadurch gekennzeichnet, dass die Verspannung des inneren im äusseren Element (22,21) formschlüssig ist, insbesondere durch Verzahnungen (22′,21′) der miteinander zur Anlage kommenden Flächen des inneren und äusseren Elementes (22,21).

4. Verbindungseinrichtung nach Anspruch 2 oder 3, dadurch gekennzeichnet, dass das innere Element (22,62) in unverspanntem Zustand längsverschiebbar und drehbar im äusseren Element (21,61) gehalten ist.

5. Verbindungseinrichtung nach Anspruch 2 oder 3, dadurch gekennzeichnet, dass das innere Element in unverspanntem Zustand in Drehrichtung bereits annähernd formschlüssig mit dem äusseren Element verbunden ist.

6. Verbindungseinrichtung nach einem der Ansprüche 1 bis 5, dadurch gekennzeichnet, dass das Verspannungsmittel eine Mutter (23,33) umfasst, welche auf ein am inneren Element (22,32) vorgesehenen Gewindeteil (29) aufschraubbar ist und dabei mit dem äusseren Element (21,31) zur Anlage kommt.

7. Verbindungseinrichtung nach einem der Ansprüche 1 bis 5, dadurch gekennzeichnet, dass das Verspannungsmittel eine Stellschraube (63) umfasst, welche in eine Gewindebohrung (69) des inneren Elementes (62) einschraubbar ist und mit einem in diesem Element (62) eingeführten Konstruktionselement (15) zur Anlage kommt, wobei letzteres stirnseitig mit dem äusseren Element (61) und dieses mit dem zweiten Konstruktionselement (16) zur Anlagekommt, welches ebenfalls im innern Element (62) eingeführt und in diesem anliegend ist.

8. Verbindungseinrichtung nach einem der vorangehenden Ansprüche, dadurch gekennzeichnet, dass die Durchgangsöffnungen (24′,25′) als Bohrungen vorgesehen sind.

9. Verbindungseinrichtung nach einem der vorangehenden Ansprüche, dadurch gekennzeichnet, dass die Durchgangsöffnungen (24,25,65) als Schlitze vorgesehen sind.

10. Verbindungseinrichtung nach einem der vorangehenden Ansprüche, dadurch gekennzeichnet, dass mindestens die eine der beiden gemeinsamen Durchgangsöffnungen (66) in den beiden Elementen (62,61) eine sektorielle Bewegung des darin eingeführten Konstruktionselementes (16) innerhalb eines Winkelbereiches gestattet.

11. Verbindungseinrichtung nach einem der vorangehenden Ansprüche, dadurch gekennzeichnet, dass wenigstens das eine innere Element (22,32,52) mit einer von seiner queren Durchgangsöffnung (24′,25′,39,55) ausgehenden, letztere sowie das zugehörige Ende (22˝,32′,52′) des inneren Elementes (22,32,52) trennende Ausnehmung (26,36,56) versehen ist, wobei das in die Durchgangsöffnungen (24′,25′,34,55) eingeführte Konstruktionselement (12,13) eine radiale Aufspreizung des geschlitzten Endes (22˝,32′,52′) des inneren Elementes (22,32,52) innerhalb der zylindrischen Bohrung des äusseren Elementes (21,31,51) bewirkt.

12. Verbindungseinrichtung nach Anspruch 11, dadurch gekennzeichnet, dass für ein äusseres Element (21) zwei identische innere Elemente (22) mit Ausnehmungen (26) vorgesehen sind.

13. Verbindungseinrichtung nach Anspruch 11 oder 12, dadurch gekennzeichnet, dass beide Durchgangsöffnungen (24,25) des äusseren Elementes (21) in Bezug auf dessen Längsachse als zueinander um 90° versetzte Schlitze ausgebildet sind, um die annähernd rechtwinklig zueinander angeordneten Konstruktionselemente (12,13) verbinden zu können.

14. Verbindungseinrichtung nach einem der Ansprüche 1 bis 10, dadurch gekennzeichnet, dass das innere Element (62,72) mit einer quer zu seiner Längsachse verlaufenden Ausnehmung (66,76) versehen ist, die zudem die Durchgangsöffnung bildet, wobei diese Ausnehmung (66,76) und das darin eingeführte Konstruktionselement (16) beim Verspannen ein radiales Verbiegen des inneren Elementes (62,72) bewirkt.

15. Verbindungseinrichtung nach Anspruch 14, dadurch gekennzeichnet, dass das innere Element (72) aus einem Gewindeteil (79), einem zylindrischen Teil (72′) mit einer queren Ausnehmung (76) und einem Wirbelhaken (75) oder einer Pedikelschraube (80) besteht, dabei das äussere Element (71) einerseits mit einer auf dem Gewindeteil (79) aufgeschraubter Mutter (73) als Verspannungsmittel und andererseits mit einem in der Ausnehmung (76) eingeführtem Konstruktionselement (13) zur Anlagekommt.

16. Verbindungseinrichtung nach Anspruch 14 oder 15, dadurch gekennzeichnet, dass die Durchgangsöffnung (55,65,74) des äusseren Elementes (51,61,71) auf dem der Ausnehmung (56,66,76) des inneren Elementes (52,62,72) zugekehrten Ende als axial verlaufende Einschlitzung ausgestaltet ist.

17. Verbindungseinrichtung nach Anspruch 6, dadurch gekennzeichnet, dass die auf das innere Element (32) aufschraubbare Mutter (33) mit einem konischen Ansatz (33′) versehen ist, welcher mit einem entsprechenden Innenkonus (38) des äusseren Elementes (31) beim Spannen in Kontakt steht.

18. Verbindungseinrichtung nach Anspruch 17, dadurch gekennzeichnet, dass der konische Ansatz (33′) mindestens einen Längsschlitz (33˝) aufweist, welcher die Selbsthemmung zwischen dem inneren und dem äusseren Element (32,31) erhöht.

19. Verbindungseinrichtung nach einem der Ansprüche 1 bis 4 oder 8 bis 10, dadurch gekennzeichnet, dass das Verspannungsmittel eine ins äussere Element (91) eingeschraubte Stellschraube (93) umfasst, die mit dem inneren Element (92) zur Anlage kommt.

20. Verbindungseinrichtung nach einem der vorangehenden Ansprüche, dadurch gekennzeichnet, dass im inneren Element (92) eine Doppelbohrung (99) als Durchgangsöffnung für ein Konstruktionselement vorgesehen ist, welche aus zwei versetzten Bohrungen gebildet ist, von denen die auf der Seite des geschlitzten Endes des inneren Elementes (92) befindliche einen kleineren Durchmesser als die andere Bohrung resp. als das eingeführte Konstruktionselement hat.

## Claims

1. Connection device for the adjustable connection of a first with a second structural component, particularly tubes or rods, for a setting device, with an external component having a cylindrical hole and at least one internal cylindrical component that can be slid at least axially in the hole, having one or preferably two through openings, both crossways to the direction of the axis, to hold a structural component, and which when inserted therein can be locked in place by means of components that can be slid axially in relation to each other by means of at least one brace, characterized by the fact that the internal component (22, 32, 52, 62, 72) that by means of the bracing element (23, 29, 33, 53, 63, 73) can be slid axially to the external component (21, 31, 51, 61, 71) has at least one recess (26, 36, 56, 66, 76), by means of which it can be flexibly deformed when slid through the inserted structural component (12, 13, 15, 16) and can thereby be additionally braced against the external component (21, 31, 51, 61, 71).

2. Connection device according to claim 1, characterized by the fact that the internal component is braced frictionally in the external component (62, 61), particularly by means of structured surfaces (67) of those surfaces of the internal and external components (62, 61) that are in contact with each other.

3. Connection device according to claim 1, characterized by the fact that the internal component is braced in the external component (22, 21) positively, particularly by denticulations (22′, 21′) of those surfaces of the internal and external components (22, 21) that are in contact with each other.

4. Connection device according to claim 2 or 3, characterized by the fact that the internal component (22, 62) is held, in unbraced condition, in the external component (21, 61) in such manner that it can slide longitudinally and can rotate.

5. Connection device according to claim 2 or 3, characterized by the fact that in unbraced state the internal component is already connected with the external component in the direction of rotation and approximately positively.

6. Connection device according to one of claims 1 to 5, characterized by the fact that the bracing medium has a nut (23, 33) that can be screwed onto a threaded segment (29) on the internal component (22, 32), and that it thereby comes into contact with the external component (21, 31).

7. Connection device according to one of claims 1 to 5, characterized by the fact that the bracing medium has a set screw (63) that can be screwed into a threaded hole (69) in internal component (62) and comes into contact with a structural component (15) introduced into this component (62), whereupon the front end of structural component (15) comes into contact with the external component (61), and the external component (61) comes into contact with the second structural component (16), which is likewise inserted into the internal component (62) and rests in said internal component (62).

8. Connection device according to one of the preceding claims, characterized by the fact that the through openings (24′, 25′) are in the form of holes.

9. Connection device according to one of the preceding claims, characterized by the fact that the through openings (24, 25, 65) have the form of slots.

10. Connection device according to one of the foregoing claims, characterized by the fact that at least one of the two common through openings (66) in the two components (62, 61) permits a sectorial movement of the structural component (16) inserted therein, within an angular range.

11. Connection device according to one of the foregoing claims, characterized by the fact that at least one internal component (22, 32, 52) has a recess (26, 36, 56) that starts from its crossways through opening (24′, 25′, 39, 55) and breaks into the said crossways through opening as well as the related end (22˝, 32′, 52′) of the internal component (22, 32, 52), with the structural component (12, 13) inserted into through openings (24′, 25′, 34, 55) creatinga radial expansion of the slotted end (22˝, 32′, 52′) of the internal component (22, 32, 52) within the cylindrical hole of the external component (21, 31, 51).

12. Connection device according to claim 11, characterized by the fact that two identical internal components (22) with recesses (26) are provided for an external component (21).

13. Connection device according to claim 11 or 12, characterized by the fact that both through openings (24, 25) of external component (21) are designed, in reference to the longitudinal axis of said external component (21), as slots offset against each other by 90°, so that they can connect the structural components (12, 13), which are positioned approximately at right angles to each other.

14. Connection device according to one of claims 1 to 10, characterized by the fact that the internal component (62, 72) has a recess (66, 76) running crossways to its longitudinal axis, which said recess also forms the through opening, with this recess (66, 76) and the structural component (16) inserted therein creating a radial bending of the internal component (62, 72) upon bracing.

15. Connection device according to claim 14, characterized by the fact that the internal component (72) consists of a threaded segment (79), a cylindrical segment (72′) with a crossways recess (76), and a swivel-hook (75) or a pedicle screw (80), with the external component (71) coming into contact on the one hand with a nut (73) screwed onto the threaded segment (70) as bracing component, and on the other hand with a structural component (13) inserted into the recess (76).

16. Connection device according to claim 14 or 15, characterized by the fact that the through opening (55, 65, 74) of the external component (51, 61, 11) on the end facing the recess (56, 66, 76) of the internal component (52, 62, 72) is designed as a slit running axially.

17. Connection device according to claim 6, characterized by the fact that the nut (33) that can be screwed onto the internal component (32) has a conical lug (33′), which when stressed is in contact with a corresponding internal cone (38) of external component (31).

18. Connection device according to claim 17, characterized by the fact that the conical lug (33′) has at least one longitudinal slot (33˝), which increases the self-retention between the internal and the external components (32, 31).

19. Connection device according to one of claims 1 to 4 or 8 to 10, characterized by the fact that the bracing medium has a set screw (93) screwed into the external component (91), which said set screw (93) comes into contact with the internal component (92).

20. Connection device according to one of the preceding claims, characterized by the fact that the internal component (92) contains a double hole (99) as through opening for the structural component, which is formed of two offset holes, the hole on the side of the slotted end of the internal component (92) having a smaller diameter than the other hole or the inserted structural component.

## Revendications

1. Dispositif de liaison pour la liaison ajustable d'un premier élément de construction avec un second élément de construction, en particulier de tubes ou de barres pour un dispositif de fixation, comportant un élément extérieur présentant un alésage cylindrique et au moins un élément cylindrique intérieur pouvant être glissé au moins axialement dans l'alésage, ces éléments cylindriques présentant une, de préférence deux ouvertures de passage commun formées transversalement par rapport à la direction axiale, pour la réception d'un élément de construction, et qui après y avoir été inséré peut être immobilisé par serrage, au moyen des éléments qui peuvent être déplacés axialement l'un par rapport à l'autre par au moins un moyen de serrage, caractérisé en ce que l'élément intérieur (22, 32, 52, 62, 72), qui peut être déplacé axialement par rapport à l'élément extérieur (21, 31, 51, 61, 71) à l'aide du moyen de serrage (23, 29, 33, 53, 63, 73), est pourvu d'au moins un évidement (26, 36, 56, 66, 76), au moyen duquel, lors du glissement, il peut être déformé élastiquement par l'élément de construction enfoncé (12, 13, 15, 16) et peut ainsi être serré en supplément contre l'élément extérieur (21, 31, 51, 61, 71).

2. Dispositif de liaison selon la revendication 1, caractérisé en ce que le serrage de l'élément intérieur dans l'élément extérieur (62, 61) est un serrage à force, en particulier au moyen de surfaces structurées (67) des surfaces de l'élément intérieur et de l'élément extérieur (62, 61) qui viennent se poser l'une contre l'autre.

3. Dispositif de liaison selon la revendication 1, caractérisé en ce que le serrage de l'élément intérieur dans l'élément extérieur (22, 21) est en correspondance de forme, en particulier par des dentelures (22′, 21′) des surfaces de l'élément intérieur et de l'élément extérieur (22, 21) qui viennent se poser l'une contre l'autre.

4. Dispositif de liaison selon la revendication 2 ou 3, caractérisé en ce que, dans son état non serré, l'élément intérieur (22, 62) est maintenu à déplacement longitudinal et à rotation dans l'élément extérieur (21, 61).

5. Dispositif de liaison selon la revendication 2 ou 3, caractérisé en ce que, dans son état non serré, l'élément intérieur est, dans le sens de la rotation, déjà relié approximativement en correspondance de forme avec l'élément extérieur.

6. Dispositif de liaison selon l'une des revendications 1 à 5, caractérisé en ce que le moyen de serrage comporte un écrou (23, 33) qui peut être vissé sur une partie filetée (29) prévue sur l'élément intérieur (22, 32), et vient ainsi se poser sur l'élément extérieur (21, 31).

7. Dispositif de liaison selon l'une des revendications 1 à 5, caractérisé en ce que le moyen de serrage comporte une vis de réglage (63), qui peut être vissée dans un alésage fileté (69) de l'élément intérieur (62) et qui vient se placer contre un élément de construction (15) enfoncé dans cet élément (62), l'élément de construction (15) venant se placer frontalement contre l'élément extérieur (61) et celui-ci venant se placer contre le second élément de construction (16), qui de même est enfoncé dans l'élément intérieur (62) et repose dans celui-ci.

8. Dispositif de liaison selon l'une des revendications précédentes, caractérisé en ce que les ouvertures de passage (24′, 25′) sont prévues comme alésages.

9. Dispositif de liaison selon l'une des revendications précédentes, caractérisé en ce que les ouvertures de passage (24, 25, 65) sont prévues comme fentes.

10. Dispositif de liaison selon l'une des revendications précédentes, caractérisé en ce qu'au moins une des deux ouvertures de passage commun (66) dans les deux éléments (62, 61) permet un déplacement sectoriel de l'élément de construction (16) qui y est enfoncé, à l'intérieur d'une plage angulaire.

11. Dispositif de liaison selon l'une des revendications précédentes, caractérisé en ce qu'au moins un des éléments intérieurs (22, 32, 52) est doté d'un évidement (26, 36, 56), qui part de son ouverture transversale de passage (24′, 25′, 39, 55) et coupe cette dernière et l'extrémité associée (22˝, 32′, 52′) de l'élément intérieur (22, 32, 52), l'élément de construction (12, 13) enfoncé dans les ouvertures de passage (24′, 25′, 34, 55) réalisant un écartement radial de l'extrémité fendue (22˝, 32′, 52′) de l'élément intérieur (22, 32, 52) à l'intérieur de l'alésage cylindrique de l'élément extérieur (21, 31, 51).

12. Dispositif de liaison selon la revendication 11, caractérisé en ce que pour un élément extérieur (21), deux éléments intérieurs identiques (22) avec évidement (26) sont prévus.

13. Dispositif de liaison selon la revendication 11 ou 12, caractérisé en ce que les deux ouvertures de passage (24, 25) de l'élément extérieur (21) sont configurées comme fentes décalées de 90° l'une par rapport l'autre suivant l'axe longitudinal de l'élément extérieur (21), pour pouvoir relier les éléments de construction (12, 13) disposés approximativement perpendiculairement l'un par rapport à l'autre.

14. Dispositif de liaison selon l'une des revendications 1 à 10, caractérisé en ce que l'élément intérieur (62, 72) est pourvu d'un évidement (66, 76) s'étendant transversalement par rapport à son axe longitudinal, qui forme à cet effet une ouverture de passage, cet évidement (66, 76) et l'élément de construction (16) qui y est enfoncé ayant pour effet une flexion axiale de l'élément intérieur (62, 72) lors du serrage.

15. Dispositif de liaison selon la revendication 14, caractérisé en ce que l'élément intérieur (72) est constitué d'une partie filetée (79), d'une pièce cylindrique (72′) comportant un évidement transversal (76), et d'un crochet vertébral (75) ou d'une vis pédiculaire (80), l'élément extérieur (71) venant se placer, d'une part, contre un écrou (73) vissé sur la partie filetée (79) et servant de moyen de serrage et, d'autre part, contre un élément de construction (13) enfoncé dans l'évidement (76).

16. Dispositif de liaison selon la revendication 14 ou 15, caractérisé en ce que l'ouverture de passage (55, 65, 74) de l'élément extérieur (51, 61, 71) est configurée comme fente s'étendant axialement sur l'extrémité de l'élément intérieur (52, 62, 72) qui est tournée vers l'évidement (56, 66, 76).

17. Dispositif de liaison selon la revendication 6, caractérisé en ce que l'écrou (33) pouvant être vissé sur l'élément intérieur (32) est pourvu d'un appendice conique (33′) qui vient se placer contre un cône intérieur (38) correspondant de l'élément extérieur (31) lors du serrage.

18. Dispositif de liaison selon la revendication 17, caractérisé en ce que l'appendice conique (33′) présente au moins une fente longitudinale (33˝), qui renforce l'autoblocage entre l'élément intérieur et l'élément extérieur (32, 31).

19. Dispositif de liaison selon l'une des revendications 1 à 4 ou 8 à 10, caractérisé en ce que le moyen de serrage comporte une vis de réglage (93) vissée dans l'élément extérieur (91) et qui vient se placer contre l'élément intérieur (92).

20. Dispositif de liaison selon l'une des revendications précédentes, caractérisé en ce que dans l'élément intérieur (92) est prévu un alésage double (99) servant d'ouverture de passage pour un élément de construction, et qui est formé de deux alésages décalés, dont celui situé sur le côté de l'extrémité fendue de l'élément intérieur (92) a un diamètre plus petit que l'autre alésage et que l'élément de construction enfoncé.
